# EUROPÄISCHE PATENTSCHRIFT

(11) **EP 4 173 662 B1**
(45) Veröffentlichungstag und Bekanntmachung des Hinweises auf die Patenterteilung: **08.10.2025**
(21) Anmeldenummer: 22203886.1
(22) Anmeldetag: 26.10.2022
(51) Int. Cl.: A61M 16/00, A61L 2/08, A61L 9/18, A61M 16/04, A61M 25/00

(54) **BEATMUNGSSYSTEM**
BREATHING SYSTEM
SYSTÈME DE VENTILATION

(30) Priorität: 26.10.2021 DE 102021127885
(43) Veröffentlichungstag der Anmeldung: 03.05.2023
(73) Patentinhaber: Technische Hochschule Ulm, 89081 Ulm (DE)
(72) Erfinder: Heßling, Martin, 89081 Ulm (DE); Hönes, Katharina, 89081 Ulm (DE); Meurle, Tobias, 89081 Ulm (DE); Knaus, Johannes, 89081 Ulm (DE); Sicks, Ben, 89081 Ulm (DE); Spellerberg, Barbara, 89081 Ulm (DE)
(74) Vertreter: Sonnenberg Harrison Partnerschaft mbB

(56) Entgegenhaltungen:
- CN-U- 204 655 574
- KR-B1- 102 213 647
- US-A1- 2007 187 626
- US-A1- 2014 378 792
- US-A1- 2015 190 649
- US-A1- 2016 317 832
- US-A1- 2019 168 023
- US-A1- 2021 106 844
- US-A1- 2021 290 974
- US-B2- 7 159 590

## Beschreibung

Die Erfindung betrifft ein Beatmungssystem wie in den angehängten Ansprüchen definiert.

Diese Anmeldung beansprucht Priorität der deutschen Patentanmeldung DE 10 2021 127 885 welche am 26. Oktober 2021 eingereicht wurde.

### Hintergrund der Erfindung

Im Bereich der Intensiv- und Notfallmedizin ist es oftmals unumgänglich, eine Patientin mittels eines Endotrachealtubus zu beatmen. Diese Endotrachealtuben umfassen üblicherweise einen dünnen, an beiden Enden geöffneten Schlauch, welcher in die Luftröhre (Trachea) der Patientin eingeführt wird. Um eine Abdichtung gegenüber der Trachea zu erreichen, sind Endotrachealtuben an dem näher an der Lunge gelegenen Ende in der Regel mit einer Blockmanschette versehen. Diese Blockmanschette wird mit einem Innendruck beaufschlagt und dichtet den Endotrachealtubus gegenüber der Trachea ab. Die Blockmanschette verhindert, dass Atemluft von der Lunge in die Trachea gelangt. Zudem verhindert die Blockmanschette, dass Körperflüssigkeiten und Krankheitserreger von der Trachea in die Lunge gelangen. Ferner stützt und fixiert die Blockmanschette den Endotrachealtubus in der Trachea und verhindert hierdurch ein ungewolltes Verrutschen oder Lösen des Endotrachealtubus in der Trachea.

Bei länger andauernden Beatmungen von Patientinnen können jedoch Keime aus bspw. der Mundflora in die Trachea gelangen. Diese Keime dringen entlang dem Endotrachealtubus bis in die Lunge vor, wodurch Pneumonien als folgenschwere Erkrankungen ausgelöst werden. Zudem kann sich innerhalb des Lumens ein mikrobieller Biofilm bilden, der gerade bei immungeschwächten Patientinnen zu schwerwiegenden Infektionen führen kann. In der Literatur sind daher verschiedene Lösungen vorbekannt, um den Endotrachealtubus auch bei länger andauernder Verwendung möglichst frei von Keimen und Krankheitserregern zu halten. Hierfür sind neben der Verwendung spezieller Materialien für den Endotrachealtubus auch verschiedene Lösungen bekannt, die eine Keimbildung durch UV-Strahlung verhindern.

Beispielsweise beschreibt die US-Patentanmeldung US 2007/187626 A1 eine Vorrichtung und ein Verfahren für das Sterilisieren medizinischer Geräte unter Anwendung von UV-Strahlung. Das System umfasst eine UV-Lichtquelle sowie eine Sterilisierungsstruktur, die für das Sterilisieren eines Hohlkörpers verwendet wird. Die Sterilisationsstruktur wird beispielsweise in den Hohlraum eines Endotrachealtubus eingeführt, um diesen von innen heraus zu sterilisieren. Die Sterilisationsstruktur ist eine längliche Struktur, die biegbar/flexibel ist. Die vorherrschende Wellenlänge einer von der UV-Lichtquelle emittierten UV-Strahlung ist so ausgewählt, dass sie mit einem Absorptionsspektren einer Ziel-DNA oder Ziel-RNA übereinstimmt, die zerstört oder inaktiviert werden soll. In einer weiteren Ausführungsform beschreibt diese US-Patentanmeldung die Verwendung mehrerer LEDs/Dioden, die in der Sterilisationsstruktur für die Inaktivierung der Ziel-DNA oder Ziel-RNA eingebracht sind. Zudem wird die Verwendung von LEDs/Dioden mit unterschiedlicher Wellenlänge für die Inaktivierung von Ziel-DNA oder Ziel-RNA mit unterschiedlichen Absorptionsspektren beschrieben.

Die internationale Patentanmeldung WO 2014/004680 A1 beschreibt ein Verfahren zur Gestaltung eines Katheters/Stents, um das Wachstum von Krebsgewebe oder Bakterien innerhalb des Katheters/Stents zu verhindern. Der Katheter/Stent kann im Rahmen einer fotodynamischen Therapie eingesetzt werden. Der Katheter/Stent enthält ein System aus mehreren eingebetteten LEDs, die das innere Lumen des Katheters/Stents beleuchten. Die LEDs sind mit einer Spannungsquelle verbunden, die sich entweder im oder außerhalb des Körpers des Patienten befindet. Die Patentanmeldung beschreibt weiter die Verwendung der LEDs für die fotodynamische Therapie, um das Lumen des Katheters/Stents von Tumorzellen und Bakterien freizuhalten. Die Leistungsabgabe der LEDs im Lumen (108) ist größer als 10 mW/cm² und die emittierte Wellenlänge der Strahlung ist im tiefroten Spektralbereich von 630 bis 780 nm. Durch die Wärmeentwicklung der LEDs wird eine geringe Temperaturerhöhung (weniger als 1 °C) ausgelöst.

Die Internationale Patentanmeldung WO 2010/023329 A1 beschreibt eine Vorrichtung und ein Verfahren für das Desinfizieren eines Anbindelements und eines Lumens medizinischer Geräte unter Anwendung von UV-Strahlung. Die Vorrichtung umfasst eine Lichtquelle. Die Lichtquelle ist beispielsweise eine UV-Lichtquelle mit einer Wellenlänge von 200 nm bis 300 nm. Durch ein optisches Fenster gelangt die UV-Strahlung von der UV-Lichtquelle in das Lumen des medizinischen Geräts. In einer weiteren Ausführungsform umfasst die Vorrichtung eine getrennte Einheit, die an einen Katheter fluiddicht angebunden ist. Die Vorrichtung wird für die Desinfektion von Endotrachealtuben verwendet, wobei für diese Desinfektion ein modifiziertes Anbindelement verwendet wird.

Die Veröffentlichungen [1] und [3] beschreiben einen selbstleuchtenden Endotrachealtubus zur Vermeidung von beatmungsassoziierten Folgeerkrankungen bei länger andauernden Beatmungen von Patientinnen. Eine Außenseite des beschriebenen selbstleuchtenden Endotrachealtubus ist mit einer Vielzahl von LEDs ausgestattet. Diese LEDs beleuchten eine Innenseite der Trachea sowie das Lumen des Endotrachealtubus mit blauem Licht. Durch Auswahl einer geeigneten Bestrahlungsdosis wird die relative Konzentration von Keimen im Lumen und in der Trachea deutlich reduziert. Aus [2] ist ein selbstleuchtender Tubus bekannt, in welchem ein violett leuchtender Lichtleiter in das Lumen des Endotrachealtubus eingeführt ist. Dieser Lichtleiter bestrahlt das Lumen des Endotrachealtubus sowie die Trachea, wobei die Bestrahlung der Trachea maßgeblich von der Lichtleitfähigkeit des ausgewählten Materials für den Endotrachealtubus abhängig ist.

Die aus den Veröffentlichungen [1] bis [3] vorbekannten Endotrachealtuben erreichen eine homogene Ausleuchtung der Trachea. Allerdings ist der in der in [1] und [2] beschriebene Endotrachealtubus aufgrund der Vielzahl der verwendeten LEDs anfällig für Kabelbrüche und Beschädigungen einzelner LEDs. Der Ausfall einzelner LEDs führt zu einer Zunahme der Spannung bzw. der Stromstärke der verbleibenden LEDs, wodurch es zu einer intensiveren Bestrahlung einzelner Bereiche der Trachea kommt. Diese intensivere Bestrahlung führt unter Umständen zu Verbrennungen der Trachea. Auch kann die Verwendung von Lichtquellen mit ungeeigneter Wellenlänge zu Beschädigungen der Trachea führen. Obwohl UV-Licht üblicherweise hervorragend zur Desinfektion geeignet ist, führt dieses UV-Licht zu einer Schädigung menschlicher Zellen und ist daher für die Verwendung in der Trachea ungeeignet. Zudem verfügt der in [1] und [2] beschriebene Endotrachealtubus über eine Vielzahl von Kabeln. Diese Kabel bergen die Gefahr, dass die Patientin bei Blankliegen des Kabels Stromschläge erleidet.

Der in [3] vorgestellte Endotrachealtubus erfordert einen in das Lumen eingebrachten Lichtleiter für die Bestrahlung der Trachea. Durch das Einbringen des Lichtleiters in das Lumen wird der für die Luftleitung verfügbare Querschnitt reduziert, wodurch eine ausreichende Sauerstoffversorgung der Patientin nicht sichergestellt ist. Daher besteht ein Bedarf, die im Stand der Technik vorbekannten Endotrachealtuben für eine langfristige und sichere Verwendung in der Patientin zu verbessern.

Weitere Lösungen sind aus den Patentdokumenten KR 102 213 647 B1, US 2015/190649 A, US 2021/106844 A1, US 2014/378792 A1, US 7 159 590 B2, CN 204 655 574 U und US 2019/168023 A1 bekannt.

Noch weitere Lösungen sind aus den Patentdokumenten US 2021/290974 A1 und US 2016/317832 A1 bekannt.

### Zusammenfassung der Erfindung

Es ist eine Aufgabe der vorliegenden Erfindung, einen Endotrachealtubus bereitzustellen, welcher die Gefährdung einer Patientin, beispielsweise durch Stromschlag oder Überschreiten einer zulässigen Bestrahlungsstärke bei einem Einsatz des Endotrachealtubus in der Trachea einer Patientin weitgehend verhindert.

Dieses Dokument lehrt ein Beatmungssystem, welches eine homogene Bestrahlung der Trachea mit sichtbarem Licht durch den Einsatz gleichmäßig aufgebrachter und durch eine Schutzschicht von der Trachea von der Patientin getrennter selbstleuchtender Oberflächen oder Lichtquellen ermöglicht. Das Beatmungssystem umfasst einen Endotrachealtubus.

Das Beatmungssystem gemäß einem ersten Aspekt umfasst einen medizinischen Schlauch, eine selbstleuchtende Oberfläche und eine Anbindeinheit. Die selbstleuchtende Oberfläche ist an einer Außenseite des medizinischen Schlauchs angeordnet und ist/wird über die Anbindeinheit mit einer Spannungsquelle elektrisch verbunden. Die selbstleuchtende Oberfläche emittiert eine Strahlung mit einer Wellenlänge von Wellenlänge von 400 nm bis 630 nm, und in einem Aspekt zwischen 400 nm und 480 nm, zum Beispiel bei 450 nm und erreicht eine Bestrahlungsstärke zwischen 1 mW/cm² und 13,4 mW/cm². Der medizinische Schlauch ist aus einem zumindest teilweise transparenten Kunststoff hergestellt. Die selbstleuchtende Oberfläche ist derart angeordnet, um auch nach innen zu leuchten und das Tubus-Lumen zu bestrahlen.

Das Beatmungssystem gemäß dem ersten Aspekt umfasst ferner eine Schutzschicht, die die selbstleuchtende Oberfläche umschließt. Die Schutzschicht ist für die von der selbstleuchtenden Oberfläche emittierte Strahlung durchlässig.

Ferner wird ein Verfahren zur Herstellung eines Beatmungssystems gemäß dem ersten Aspekt oder eines Beatmungssystems gemäß dem zweiten Aspekt offenbart. Das Verfahren umfasst das Bereitstellen eines medizinischen Schlauchs, das Anordnen der selbstleuchtenden Oberfläche oder der Lichtquelle an einer Außenseite des medizinischen Schlauchs. Zudem umfasst das Verfahren das Anbringen einer Schutzschicht über der selbstleuchtenden Oberfläche oder der Lichtquelle.

### Beschreibung der Figuren

Fig. 1 zeigt ein Beatmungssystem mit einem ersten Aspekt eines Endotrachealtubus.
Fig. 2 zeigt eine Detailansicht des ersten Aspekts des Endotrachealtubus.
Fig. 3 zeigt das Beatmungssystem mit einem zweiten Aspekt des Endotrachealtubus.
Fig. 4 zeigt eine Detailansicht des zweiten Aspekts des Endotrachealtubus.
Fig. 5 zeigt ein Verfahren zur Herstellung des Beatmungssystems gemäß dem ersten Aspekt oder des Beatmungssystems gemäß dem zweiten Aspekt.
Fig. 6 zeigt einen dritten Aspekt des Beatmungssystems mit dem Endotrachealtubus nach dem ersten oder zweiten Aspekt.

### Detaillierte Beschreibung der Erfindung

Die Offenbarung wird nun auf der Grundlage der Zeichnungen beschrieben. Es wird davon ausgegangen, dass die hier beschriebenen Ausführungsformen und Aspekte der Offenbarung nur Beispiele sind und den Schutzumfang der Ansprüche in keiner Weise einschränken. Die Erfindung wird durch die Ansprüche und deren Äquivalente definiert.

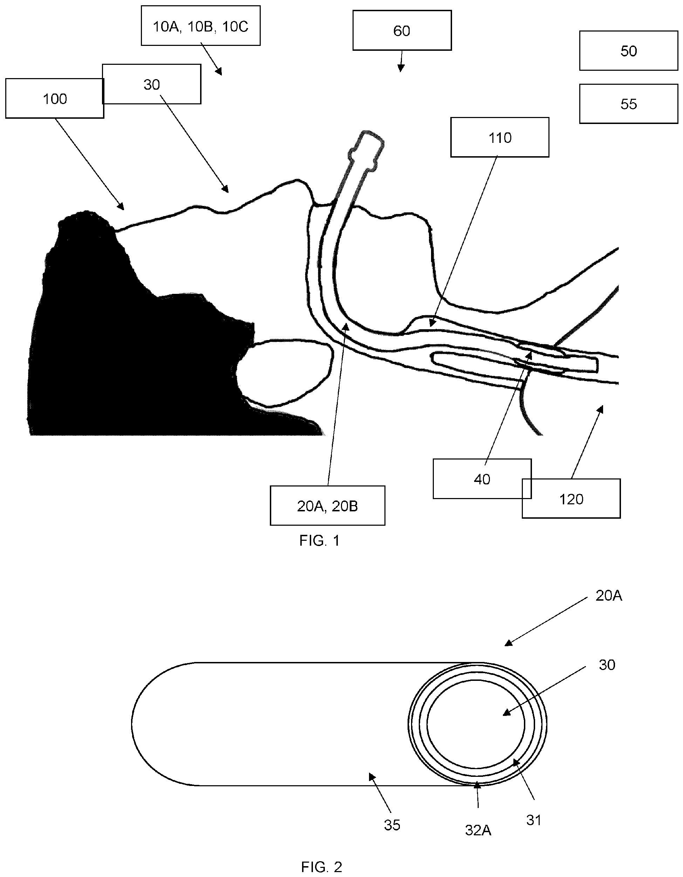

Fig. 1 zeigt ein Beatmungssystem 10A mit einem ersten Aspekt eines Endotrachealtubus 20A. Das Beatmungssystem 10A umfasst den Endotrachealtubus 20A, eine Anbindeinheit 60, eine Spannungsquelle 50, und optional eine Steuereinheit 55. Der Endotrachealtubus 20 wird in die Trachea 110 einer Patientin 100 eingebracht. Der Endotrachealtubus 20 schafft eine Fluidverbindung zwischen der Anbindeinheit 60 und einer Lunge 120 der Patientin 100. Der Endotrachealtubus 20A wird über eine Manschette 40 gegenüber der Trachea 110 fluiddicht abgedichtet. Die Manschette 40 wird durch einen Luftschlauch (nicht dargestellt) aufgepumpt.

Fig. 2 zeigt eine Detailansicht des ersten Aspekts des Endotrachealtubus 20A. Der Endotrachealtubus 20A umfasst einen medizinischen Schlauch 31, eine selbstleuchtende Oberfläche 32A, und eine Schutzschicht 35. Der medizinische Schlauch 31 ist ein länglicher Schlauch und weist einen im Wesentlichen runden Querschnitt auf. Der medizinische Schlauch 31 formt einen Innenraum welcher als Lumen 30 bezeichnet wird. Der medizinische Schlauch 31 ist auf einer Außenseite, welche auf der dem Innenraum gegenüberliegenden Seite angeordnet ist, flächig von einer selbstleuchtenden Oberfläche 32A umgeben bzw. ummantelt. Durch das flächige Aufbringen der selbstleuchtenden Oberfläche 32A wird eine weitgehend homogene Bestrahlung der Trachea 110 ermöglicht. Die Risiken eines lokalen Überschreitens einer zulässigen Bestrahlungsstärke wird durch die homogene Bestrahlung der Trachea 110 reduziert. Eine Gefährdung der Patientin 110 durch das Überschreiten der zulässigen Bestrahlungsstärke wird somit weitgehend verhindert. Der medizinische Schlauch 31 und die selbstleuchtende Oberfläche 32A sind im Wesentlichen konzentrisch angeordnet. Die selbstleuchtende Oberfläche 32A ist auf einer Außenseite, welche den medizinischen Schlauch 31 nicht berührt, flächig von einer Schutzschicht 35 umgeben. Die Schutzschicht 35 ist im Wesentlichen konzentrisch zur Lichtquelle 32 angeordnet.

Der medizinische Schlauch 31 ist aus einem zumindest teilweise transparenten Kunststoff hergestellt. Der Kunststoff ist biegbar und lokal reversibel verformbar. Der Kunststoff ist beispielsweise ein Polymer wie Polyurethan oder Polyvinylchlorid. Die Transparenz ermöglicht die Durchstrahlung des medizinischen Schlauchs, damit man Innen- und Außenbereich beleuchten kann. Für den Ansatz, dass der Schlauch auch direkt Lichtleiter ist, ist sogar eine hohe Transparenz notwendig, bzw. am unteren Ende sollte der Schlauch rau sein, oder Streukörper enthalten, damit das Licht zur Seite emittiert wird.

Der medizinische Schlauch 31 formt das Lumen 30. Das Lumen 30 wird für die Leitung des Fluids von der Anbindeinheit 60 zu der Lunge 120 der Patientin 100 verwendet. Das Fluid ist beispielsweise Umgebungsluft oder Luft mit erhöhtem Sauerstoffgehalt, wie sie für eine Beatmung der Patientin 100 eingesetzt wird. Das Fluid kann ferner weitere Zusatzstoffe umfassen, wenn diese Zusatzstoffe dem Fluid zu therapeutischen Zwecken beigefügt werden.

Die selbstleuchtende Oberfläche 32A umfasst in diesem ersten Aspekt des Endotrachealtubus 20A beispielsweise organische Leuchtdioden (OLEDs) oder ähnliche Lichtelemente wie PLED und AMOLED. Die selbstleuchtende Oberfläche 32A emittiert eine Strahlung mit einer Wellenlänge von 400 bis 630 nm, und in einem Aspekt zwischen 400 nm und 480 nm, zum Beispiel 450 nm. Die Wellenlänge der selbstleuchtenden Oberfläche 32A ist so ausgewählt, dass durch die emittierte Strahlung eine Beschädigung der Trachea weitgehend vermieden ist. In einem Aspekt wird der violette und blaue Spektralbereich von 400 bis 480 nm verwendet, dabei ist die antimikrobielle Strahlung um 410 nm ca. 5x stärker als um 450 nm.

Es sind auch noch weitere Wellenlängen denkbar, bei denen endogene Photosensitizer wie Porphyrine absorbieren, wie z.B. Protoporphyrin IX-Absorptionen bei ca. 520 nm (grün), 540 nm (grün), 575 nm (gelb) und 630 nm (rot). Diese Wellenlängen wirken zwar weniger antimikrobiell, aber auch weniger belastend auf menschliche Zellen. Auch eine Kombination von mehreren dieser Wellenlängen ist denkbar bis hin zu weißem Licht.

Die Verwendung von Leuchtelementen in einem UVA-Bereich unterhalb von 400 nm wäre auch denkbar. In diesem UVA-Bereich geht es nicht um DNA-Schädigung der menschlichen Zellen, sondern um andere photochemische Prozesse bzw. auch Wirkungsmechanismen, die über Photosensitizer ablaufen. Es könnte daher sein, dass die Strahlung in diesem UVA Bereich weniger belastend für die menschlichen Zellen sein.

Die selbstleuchtende Oberfläche 32A erreicht eine Bestrahlungsstärke zwischen 1 und 70 mW/cm² z.B. 3,1 mW/cm², wobei mit dieser selbstleuchtenden Oberfläche 32A auch Bestrahlungsstärken bis 13,4 mW/cm² erreicht werden können. Der menschliche Körper hält 1000-1300 W/cm² durch Sonnenlicht im Sommer aus und daher können theoretische auch stärkere Leuchtelemente verwendet werden.

Es könnte sein, dass sich in der Praxis herausstellt, dass auch eine deutlich geringe Bestrahlung, sogar bei 0,1 mW/cm² ausreicht, um eine Vermehrung der Keime zu verhindern.

Es ist auch denkbar, dass die Bestrahlung nicht schwach und kontinuierlich durchgeführt wird, sondern diskontinuierlich mit einer entsprechend hohen Bestrahlungsstärke (für eine kurze Zeit) und dann in Bereich von 100mW/cm2 ("entspricht Sommersonne") oder darüber.

Die selbstleuchtende Oberfläche 32A wird von der Spanungsquelle 50 mit elektrischer Energie versorgt. Die Spanungsquelle 50 wird optional von einer Steuereinheit 55 gesteuert. Die Spannungsquelle 50 versorgt die selbstleuchtende Oberfläche 32A mit einer Spannung von z.B. 3V und einer Stromstärke von z.B. 10 mA.

Die Schutzschicht 35 besteht aus einem lichtdurchlässigen Kunststoff oder einem Kunststoff mit gewisser Teilreflexion. Der lichtdurchlässige Kunststoff ist biokompatibel und ist beispielsweise Polycarbonat. Die Schutzschicht 35 schützt die Patientin 100 vor einem elektrischen Kontakt zwischen elektrischen Anschlüssen der selbstleuchtenden Oberfläche 32A und der Trachea 110. Eine Gefährdung der Patientin durch Stromschlag wird durch die Schutzschicht 35 verhindert. Die von der selbstleuchtende Oberfläche 32A emittierte Strahlung passiert die Schutzschicht 35 im Wesentlichen ohne Leistungsverlust. Die emittierte Strahlung töte Keime und Bakterien auf einer Außenseite der Schutzschicht 35 sowie auf der Innenseite der Trachea 110 ab.

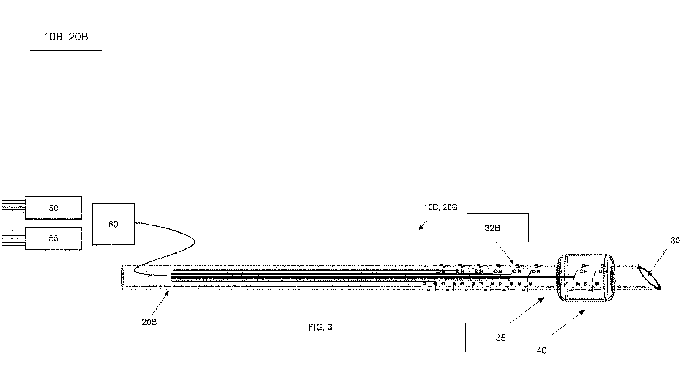

Fig. 3 zeigt ein Beatmungssystem 10B mit einem zweiten Aspekt des Endotrachealtubus 20B. Der Endotrachealtubus 20B des zweiten Aspekts ist im Wesentlichen gleich zu dem Endotrachealtubus 20A des ersten Aspekts. Die Merkmale des Endotrachealtubus 20B des zweiten Aspekts, die mit den Merkmalen des Endotrachealtubus 20A des ersten Aspekts im Wesentlichen übereinstimmen, werden der Kürze halber nicht erneut beschrieben. Für diese gleichen Merkmale werden dieselben Bezugszeichen wie die des ersten Aspekts verwendet.

Allerdings verfügt der Endotrachealtubus 20B des zweiten Aspekts nicht über eine selbstleuchtende Oberfläche 32A, sondern über zumindest eine Lichtquelle 32B. Die Lichtquelle 32B ist z.B. einen Lichtleiter und spiralförmig an der Außenseite des medizinischen Schlauchs 31 angeordnet.

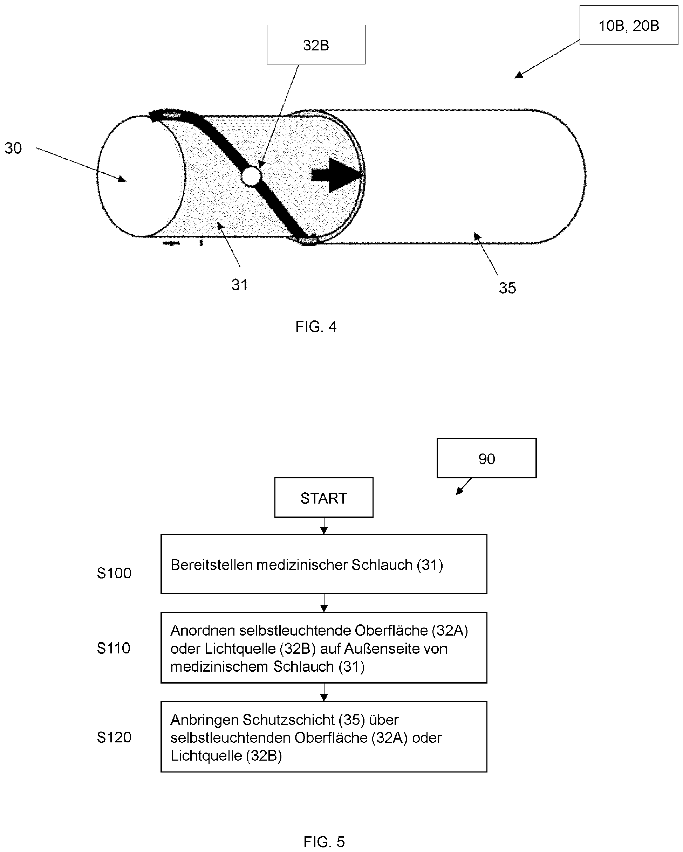

Fig. 4 zeigt eine Detailansicht des zweiten Aspekts des Endotrachealtubus 20B. Die Lichtquelle 32B ist in diesem zweiten Aspekt des Endotrachealtubus 20B ebenfalls von der Schutzschicht 35 umgegeben. Die Schutzschicht 35 schützt die Patientin 100 vor einem elektrischen Kontakt zwischen einem Leitungsdraht der Lichtquelle 32B und der Trachea 110. Eine Gefährdung der Patientin durch Stromschlag wird durch die Schutzschicht 35 verhindert. Die Lichtquelle 32B umfasst in diesem zweiten Aspekt eine Vielzahl von Leuchtdioden (LEDs), die auf einem Leitungsdraht in einem gewissen Abstand zueinander, beispielsweise 1 cm, aufgebracht sind/werden. Durch das Aufbringen der LEDs auf dem Leitungsdraht wird eine homogene Bestrahlung der Trachea 110 weitgehend ermöglicht. Das Risiko eines lokalen Überschreitens einer zulässigen Bestrahlungsstärke wird durch die homogene Bestrahlung der Trachea 110 reduziert. Eine Gefährdung der Patientin 110 durch das Überschreiten der zulässigen Bestrahlungsstärke wird somit weitgehend verhindert. Die Vielzahl von Leuchtdioden sind identische Leuchtdioden oder identische Gruppen von Leuchtdioden. In einem weiteren Beispiel sind die Leuchtdioden unterschiedliche Leuchtdioden. Als Leuchtdioden kommen die obengenannten Leuchtdioden mit unterschiedlichen Wellenlängen in Frage.

Die Lichtquelle 32B des zweiten Aspekts erreicht auch eine Bestrahlungsstärke von ähnlichen Werten wie in dem ersten Aspekt.

In einem weiteren Aspekt sind mehrere Lichtquellen 32B spiralförmig an der Außenseite des medizinischen Schlauchs 31 in einem Lichtleichter angeordnet. Diese Lichtquellen 32B sind parallel zueinander entlang der Außenseite des medizinischen Schlauchs 31 angeordnet. In einem weiteren Beispiel sind die Lichtquellen 32B ein einer sich überkreuzenden Weise entlang der Außenseite des medizinischen Schlauchs 31 angeordnet. In einem weiteren Aspekt sind die Lichtquellen 32B fluiddicht verdrahtet, wobei in diesem weiteren Aspekt die Schutzschicht 35 entfällt. Der Lichtleiter kann auch in der Tubuswand eingebettet werden.

Grundsätzlich könnte der Lichtleiter am Tubusboden liegen und eventuell in der Flüssigkeit. Bei dieser Anordnung innerhalb des Tubus funktioniert die Totalreflexion im Lichtleiter möglicherweise nicht mehr und das Licht könnte an der falschen Stelle austreten. Durch die Verlegung des Lichtleiters außerhalb des Tubus ist so mehr "Platz" für den Atemstrom innerhalb des Tubus und für medizinische Proben, um Lungengewebe oder Flüssigkeiten oder sonstige Proben aus der Lunge zu entnehmen.

Die Lichtquellen 32A und 32B können nicht (nur) nach außen leuchten, sondern auch nach innen. Damit wird zum einen das Tubus-Lumen bestrahlt und zum anderen die Bestrahlung (aufgrund der Abstrahlwinkel) homogener (d.h. keine punktuelle Bestrahlung).

Fig. 5 zeigt ein Verfahren zur Herstellung zur Herstellung von dem Beatmungssystem 10A gemäß dem ersten Aspekt oder einem Beatmungssystem 10B gemäß dem zweiten Aspekt. Das Verfahren 90 umfasst das Bereitstellen S100 des medizinischen Schlauchs 31 in Schritt S100. Das Verfahren 90 umfasst weiter das Anordnen der selbstleuchtenden Oberfläche 32A oder der Lichtquelle 32B an der Außenseite des medizinischen Schlauchs 31 in Schritt S110. Das Verfahren 90 umfasst zudem das Anbringen der Schutzschicht 35 über der selbstleuchtenden Oberfläche 32A oder der Lichtquelle 32B in Schritt S120.

Weitere Versuche haben gezeigt, dass es im Bereich der Manschette 40 des Endotrachealtubus 20A, 20B zu einer Medienansammlung kommen kann. Dieses Medium beinhaltet beispielsweise Körpersekrete wie Speichel, Blut oder weitere im Körper vorkommende Flüssigkeiten. Bei einer starken Medienansammlung kann es jedoch dazu kommen, dass lokal im Bereich der Medienansammlung eine unzureichende Abtötung der Keime und Bakterien auf einer Außenseite der Schutzschicht 35 sowie auf der Innenseite der Trachea 110 erfolgt.

Diese unzureichende Abtötung lässt sich unter anderem darauf zurückführen, dass menschliches Blut beziehungsweise Hämoglobin ein Absorptionsmaximum für Strahlung einer Wellenlänge von 410 nm aufweist. Demnach wird durch das menschliche Blut das für die Bestrahlung verwendete sichtbare Licht in Wellenlängenbereichen um 410 nm beispielsweise stark absorbiert. Dieses Auftreten des Absorptionsmaximums kann durch Verwendung von sichtbarem Licht mit Wellenlängen beispielsweise im Bereich von 450 nm (blau) reduziert werden. Beispielsweise kann es ferner vorteilhaft sein, die Bestrahlungsstärke gezielt an ein Auftreten der Medienansammlung im Bereich der Manschette 40 anzupassen.

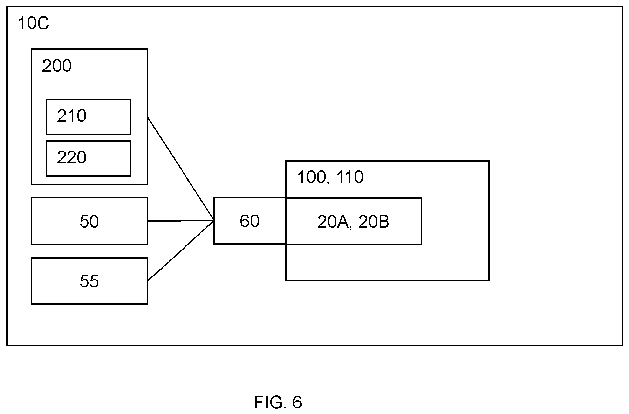

Hierfür zeigt Fig. 6 eine schematische Darstellung eines Beatmungssystem 10C nach einem dritten Aspekt. Das Beatmungssystem 10C nach diesem dritten Aspekt umfasst im Wesentlichen die Komponenten des Beatmungssystems 10A nach dem ersten Aspekt oder des Beatmungssystems 10B nach dem zweiten Aspekt. Das Beatmungssystem 10C umfasst demnach den Endotrachealtubus 20A nach dem ersten Aspekt oder den Endotrachealtubus 20B nach dem zweiten Aspekt. Der Endotrachealtubus 20A nach dem ersten Aspekt weist die selbstleuchtenden Oberfläche 32A für die Bestrahlung der Trachea 110 auf. Der Endotrachealtubus 20B nach dem zweiten Aspekt weist die Lichtquelle 32B für die Bestrahlung der Trachea 110 auf.

Demnach umfasst das Beatmungssystem 10C den Endotrachealtubus 20A, 20B umfassend den medizinischen Schlauch 31, mindestens eine von der selbstleuchtenden Oberfläche 32A oder der zumindest einen Lichtquelle 32B. Ferner umfasst das Beatmungssystem 10C die Anbindeinheit 60 und eine Analyseeinheit 200.

Die Analyseeinheit 200 umfasst eine Lichtquelle 210 und eine Erfassungseinheit 220. Die Lichtquelle 210 ist beispielsweise eine Laserlichtquelle oder eine Lichtquelle für sichtbares Licht. Die Lichtquelle für sichtbares Licht kann beispielsweise identisch mit der selbstleuchtenden Oberfläche 32A oder der Lichtquelle 32B sein. Die Laserlichtquelle ist beispielsweise über die Anbindeinheit 60 mit einem in dem Endotrachealtubus 20A, 20B angeordneten zweiten Lichtleiter verbunden beziehungsweise optisch angebunden. Der zweite Lichtleiter kann im Wesentlichen dem Lichtleiter nach dem ersten Aspekt des Endotrachealtubus 20A entsprechen. Allerdings ist der zweite Lichtleiter mit der Lichtquelle 210 verbunden. Beispielsweise kann jedoch der zweite Lichtleiter auch in dem Lumen 30 des Endotrachealtubus verlaufen.

Der zweite Lichtleiter erstreckt sich entlang des Endotrachealtubus 20A, 20B und strahlt Licht nach außen in die Trachea 110 der Patientin 100 ab. Die Lichtquelle 210 ist eine spektral breitbandige Lichtquelle wie beispielsweise eine weiße LED des Typs "Seoul Sunlike" mit einem Spektrum von 400 - 750 nm. Das von der Lichtquelle 210 emittierte Licht wird beispielsweise über die Anbindeinheit 60 in den zweiten Lichtleiter eingekoppelt. Der zweite Lichtleiter strahlt das eingekoppelte Licht in die Trachea 110 der Patientin ab und empfängt das von der Trachea 110 der Patientin 100 reflektierte Licht.

Die Erfassungseinheit 210 ist ebenfalls mit dem zweiten Lichtleiter gekoppelt und erfasst das von der Trachea 110 reflektierte und von dem zweiten Lichtleiter transportierte reflektierte Licht. Die Erfassungseinheit 210 umfasst beispielsweise ein Spektrometer. Das Spektrometer ist eingerichtet, um ein Spektrum des von der Trachea 110 reflektierten und von dem zweiten Lichtleiter transportierten reflektierten Lichts auszuwerten.

Diese Auswertung des Spektrums ermöglicht es, Rückschlüsse auf die Zusammensetzung des den Endotrachealtubus 20A, 20B umgebenden Mediums beziehungsweise Sekret zu ziehen. Beispielsweise wird es durch die Auswertung des Spektrums möglich, einen Blutanteil in dem Medium zu prognostizieren. Blut beziehungsweise Hämoglobin weisen eine deutliche spektrale Absorption von Lichtwellen mit einer Wellenlänge von 410 nm auf. Demnach ist es möglich, aus einer zunehmenden Absorption des Spektralbereiches der Wellenlänge 410 nm auf eine steigende Blutkonzentration zu schließen.

Ferner lässt sich eine steigende Bakterienkonzentration in dem Medium durch eine Zunahme der Absorption von Lichtwellen mit höheren Wellenlängen erkennen. Das bei der Bestrahlung der Trachea 110 und des Mediums verwendete Licht der Wellenläge 405 nm bis 450 nm eignet sich für eine Fluoreszenzanregung von in dem Medium enthaltenen Photosensitizern wie beispielsweise Porphyrinen oder Flavinen. Diese Photosensitzer sind in den Bakterien des Mediums enthalten und ermöglichen demnach Rückschlüsse auf das Vorhandensein der Bakterien. Beispielsweise reagieren Porphyrine auf die Bestrahlung mit einer Fluoreszenzabstrahlung mit einer Wellenlänge von etwa 630 nm. Beispielsweise reagieren Flavine auf die Bestrahlung mit einer Fluoreszenzabstrahlung mit einer Wellenlänge von etwa 530 nm. Demnach ist es möglich, aus der Erfassung der Fluoreszenzabstrahlung in Reaktion auf das durch den zweiten Lichtleiter in die Trachea 110 eingekoppelte Licht die Bakterienkonzentration in dem Medium zu ermitteln.

Durch die Erfassung der Blut- und/oder Bakterienkonzentration in dem Medium wird es zudem möglich, die Bestrahlungsstärke des Endotrachealtubus 20A, 20B gezielt anzupassen. Beispielsweise ist es möglich, bei einer Zunahme der Blut- und/oder Bakterienkonzentration die Bestrahlungsstärke kontinuierlich oder schrittweise zu erhöhen, um eine effiziente Bestrahlung der Trachea 110 sicherzustellen. Hierdurch kann die Keimbildung gezielt verhindert werden.

### Literaturverzeichnis

[1] Hessling, Martin & Hönes, Katharina & Meurle, Tobias & Knaus, Johannes & Sicks, Ben & Bauer, Richard & Spellerberg, Barbara. (2021). Leuchtende, LED-basierte Endotrachealtuben gegen Pneumonien. 10.13140/RG.2.2.25292.54403.
[2] Meurle, Tobias & Knaus, Johannes & Barbano, Agustin & Hönes, Katharina & Spellerberg, Barbara & Hessling, Martin. (2021). Photoinactivation of Staphylococci with 405 nm Light in a Trachea Model with Saliva Substitute at 37 °C. Healthcare. 9. 310. 10.3390/healthcare9030310.
[3] Sicks, Ben & Hönes, Katharina & Spellerberg, Barbara & Hessling, Martin. (2020). Blue LEDs in Endotracheal Tubes May Prevent Ventilator-Associated Pneumonia. Photobiomodulation, Photomedicine, and Laser Surgery. 38. 571-576. 10.1089/photob.2020.4842.

### Bezugszeichenliste

- 10A, 10B, 10C: Beatmungssystem
- 20A, 20B: Endotrachealtubus
- 30: Lumen
- 31: medizinischer Schlauch
- 32A: selbstleuchtende Oberfläche
- 32B: Lichtquelle
- 35: Schutzschicht
- 40: Manschette
- 50: Spanungsquelle
- 55: Steuereinheit
- 60: Anbindeinheit
- 100: Patientin
- 110: Trachea
- 120: Lunge
- 200: Analyseeinheit

## Patentansprüche

1. Beatmungssystem (10A) umfassend einen Endotrachealtubus (20A), wobei der Endotrachealtubus (20A) umfasst:
einen medizinischen Schlauch (31);
eine selbstleuchtende Oberfläche (32A) für eine Bestrahlung einer Trachea (110) einer Patientin (100), wobei die selbstleuchtende Oberfläche (32A) an einer Außenseite des medizinischen Schlauchs (31) angeordnet ist, wobei die selbstleuchtende Oberfläche (32A) zumindest eines von einer OLED, einer PLED, oder einer AMOLED umfasst, wobei die selbstleuchtende Oberfläche (32A) eine Strahlung mit einer Wellenlänge von 400 nm bis 630 nm emittiert, und wobei die selbstleuchtende Oberfläche (32A) eine Bestrahlungsstärke zwischen 1 mW/cm² und 13,4 mW/cm² erreicht; und
eine Anbindeinheit (60), wobei die selbstleuchtende Oberfläche (32A) über die Anbindeinheit (60) mit einer Spannungsquelle (50) elektrisch verbunden ist/wird,
**dadurch gekennzeichnet, dass** der medizinische Schlauch (31) aus einem zumindest teilweise transparenten Kunststoff hergestellt ist, und
dass die selbstleuchtende Oberfläche (32A) derart angeordnet ist, um auch nach innen zu leuchten und das Tubus-Lumen zu bestrahlen.

2. Beatmungssystem (10A) gemäß Anspruch 1, wobei das Beatmungssystem (10A) weiter umfasst:
eine Schutzschicht (35), die die selbstleuchtende Oberfläche (32A) umschließt.

3. Beatmungssystem (10A) gemäß Anspruch 1 oder 2, wobei
die Schutzschicht (35) für die von der selbstleuchtenden Oberfläche (32A) emittierte Strahlung durchlässig ist.

4. Beatmungssystem (10C) gemäß einem der Ansprüche 1 bis 3, ferner umfassend:
eine Analyseeinheit (200) zum Auswerten eines von einer Trachea reflektierten Lichts.

5. Verfahren (90) zur Herstellung von einem Beatmungssystem (10A, 10C) nach einem der Ansprüche 1 bis 4,
wobei das Verfahren (90) umfasst:
Bereitstellen (S100) eines medizinischen Schlauchs (31); und
Anordnen (S110) der selbstleuchtenden Oberfläche (32A) oder der Lichtquelle (32B) an einer Außenseite des medizinischen Schlauchs (31).

6. Verfahren (90) gemäß Anspruch 5, weiter umfassend:
Anbringen (S120) einer Schutzschicht (35) über der selbstleuchtenden Oberfläche (32A) oder der Lichtquelle (32B).

## Claims

1. A ventilation system (10A) comprising an endotracheal tube (20A), the endotracheal tube (20A) comprising:
a medical tube (31);
a self-illuminating surface (32A) for irradiating a trachea (110) of a patient (100), wherein the self-illuminating surface (32A) is disposed on an outside of the medical tube (31), wherein the self-illuminating surface (32A) comprises at least one of an OLED, a PLED, or an AMOLED, wherein the self-illuminating surface (32A) emits radiation having a wavelength of 400 nm to 630 nm, and wherein the self-illuminating surface (32A) reaches an irradiance of between 1 mW/cm² and 13.4 mW/cm² ; and
a connection unit (60), wherein the self-illuminating surface (32A) is electrically connected to a power source (50) via the connection unit (60),
**characterized in that** the medical tube (31) is made of an at least partially transparent plastic, and
that the self-illuminating surface (32A) is disposed so as to also illuminate inwardly and irradiate the tube lumen.

2. The ventilation system (10A) according to claim 1, wherein the ventilation system (10A) further comprises:
a protective layer (35) enclosing the self-illuminating surface (32A).

3. The ventilation system (10A) according to claim 1 or 2, wherein
the protective layer (35) is transparent to the radiation emitted from the self-illuminating surface (32A).

4. The ventilation system (10C) according to any one of claims 1 to 3, further comprising:
an analysis unit (200) for evaluating a light reflected from a trachea.

5. A method (90) for manufacturing a ventilation system (10A 10C) according to any one of claims 1 to 4, the method (90) comprising:
providing (S100) a medical tube (31); and
arranging (S110) the self-illuminating surface (32A) or the light source (32B) on an outside of the medical tube (31).

6. The method (90) according to claim 5, further comprising:
disposing (S120) a protective layer (35) over the self-illuminating surface (32A) or the light source (32B).

## Revendications

1. Système respiratoire (10A) comprenant un tube endotrachéal (20A), le tube endotrachéal (20A) comprenant :
un tube médical (31) ;
une surface auto-éclairante (32A) pour irradier la trachée (110) d'une patiente (100), la surface auto-éclairante (32A) étant disposée à l'extérieur du tube médical (31), la surface auto-éclairante (32A) comprenant au moins l'une d'une OLED, d'une PLED ou d'une AMOLED, la surface auto-éclairante (32A) émettant un rayonnement ayant une longueur d'onde de 400 nm à 630 nm, et la surface auto-éclairante (32A) atteignant une puissance d'irradiation comprise entre 1 mW/cm² et 13,4 mW/cm² ; et une unité de connexion (60), la surface auto-éclairante (32A) étant connectée électriquement à une source de tension (50) par l'intermédiaire de l'unité de connexion (60),
**caractérisé en ce que** le tube médical (31) est fabriqué à partir d'une matière plastique au moins partiellement transparente, et
**en ce que** la surface auto-éclairante (32A) est disposée pour éclairer également vers l'intérieur et irradier la lumière du tube.

2. Système respiratoire (10A) selon la revendication 1, le système respiratoire (10A) comprenant en outre :
une couche protectrice (35) entourant la surface auto-éclairante (32A).

3. Système respiratoire (10A) selon la revendication 1 ou 2, dans lequel
la couche protectrice (35) est transparente au rayonnement émis par la surface auto-éclairante (32A).

4. Système respiratoire (10C) selon l'une quelconque des revendications 1 à 3, comprenant en outre :
une unité d'analyse (200) pour évaluer la lumière réfléchie par une trachée.

5. Procédé (90) de fabrication d'un système respiratoire (10A, 10C) selon l'une quelconque des revendications 1 à 4, le procédé (90) comprenant :
fournir (S100) un tube médical (31) ; et
disposer (S110) la surface auto-éclairante (32A) ou la source de lumière (32B) sur un extérieur du tube médical (31).

6. Procédé (90) selon la revendication 5, comprenant en outre :
disposer (S120) une couche protectrice (35) sur la surface auto-éclairante (32A) ou la source de lumière (32B).
